# DEMANDE DE BREVET EUROPEEN

(11) **EP 2 851 077 A1**
(43) Date de publication de la demande: **25.03.2015**
(21) Numéro de dépôt: 14191724.5
(22) Date de dépôt: 12.04.2010
(51) Int. Cl.: A61K 33/00, A61P 25/04, A61P 25/06, A61P 29/00, A61P 29/02, A61P 21/00, A61P 19/02, A61P 25/00, A61K 9/00, A61K 45/06

(54) **Utilisation de protoxyde d'azote pour le traitement d'une douleur chronique d'origine dysfunctionnelle**

(30) Priorité: 29.04.2009 FR 0952807
(62) Demande divisionnaire de: 10723653.1
(71) Demandeur: L'AIR LIQUIDE, SOCIETE ANONYME POUR L'ETUDE ET L'EXPLOITATION DES PROCEDES GEORGES CLAUDE, 75007 Paris (FR)
(72) Inventeur: Simonnet, Guy, 33200 BORDEAUX (FR); Bessiere, Baptiste, 92130 ISSY LES MOULINEAUX (FR)
(74) Mandataire: Pittis, Olivier

(57) **Abrégé**

L'invention porte sur un mélange gazeux contenant une proportion de protoxyde d'azote (N₂O) comprise entre 15 et 45 % en volume pour une utilisation en tant que médicament inhalable pour le traitement curatif d'une douleur chronique d'origine dysfonctionnelle chez un mammifère, par réduction de l'hypersensibilité à la douleur choisie parmi les allodynies et les hyperalgésies. La douleur d'origine dysfonctionnelle peut être liée à une fibromyalgie, au syndrome du côlon irritable ou à une céphalée.

## Description

L'invention concerne l'utilisation de protoxyde d'azote (N₂O) à faibles concentrations, typiquement à moins de 50% en volume, dans le traitement des douleurs chroniques d'origine dysfonctionnelle.

Dans la population générale, on estime qu'une personne sur six souffre de douleurs chroniques, comme décrit par le document Breivik,H. et al, Survey of chronic pain in Europe: prevalence, impact on daily life, and treatment. Eur. J. Pain 10, 287-333 (2006).

Parmi les douleurs chroniques, les douleurs neuropathiques sont considérées comme les plus sévères et les plus persistantes.

Les douleurs neuropathiques surviennent à la suite d'une lésion ou d'un dysfonctionnement du système nerveux qui peut être :
- soit d'origine périphérique, par exemple une lésion ou une irritation d'origine traumatique, toxique, métabolique, ischémique, immuno-allergique ou infectieuse des nerfs périphériques,
- soit d'origine centrale, par exemple une lésion ou une irritation de la moelle épinière ou de l'encéphale.

Les douleurs neuropathiques ne répondent pas aux antalgiques usuels, tel que anti inflammatoires non stéroïdiens, paracétamol ou composés salicylés, et sont souvent peu sensibles aux analgésiques opioïdes.

Au niveau neurobiologique, l'hypersensibilité à la douleur, c'est-à-dire l'hyperalgésie ou l'allodynie, consécutive à une lésion nerveuse (douleur neuropathique) ne relève pas seulement d'un excès de nociception mais fait intervenir des processus de sensibilisation à la douleur, comme rappelé par les documents T.J. Coderre, et al. Perpheral and central hyperexcitability: differential signs and symptoms in persistent pain, Behav. Brain Sci. 20, 404-419 (1997); et C.J. Woolf et al, Neuropathic pain: aetiology, symptoms, mechanisms, and management, Lancet 353, 1959-1964 (1999).

Le rôle clef joué par le récepteur NMDA (N-methyl-D-aspartate) dans le processus de sensibilisation à la douleur fait de ce récepteur une cible thérapeutique de choix dans le traitement des douleurs chroniques principalement d'origine neuropathique, comme exposé par le document C.G. Parsons. NMDA receptors as targets for drug action in neuropathic pain, Eur. J. Pharmacol. 429, 71-78 (2001). Ainsi, l'efficacité clinique des antagonistes des récepteurs NMDA, tel que kétamine, amantadine ou mémantine, a été décrite, notamment par le document Chizh,B.A. et al, NMDA antagonists and neuropathic pain--multiple drug targets and multiple uses, Curr. Pharm. Des 11, 2977-2994 (2005).

Cependant, les effets indésirables des antagonistes des récepteurs NMDA actuels, tels que des troubles de la mémoire, des effets psychodysleptiques, une ataxie et une mauvaise coordination motrice contrebalancent largement les bénéfices attendus et en interdisent une utilisation large ou prolongée, comme expliqué par les documents J.A. Kemp et al, NMDA receptor pathways as drug targets, Nat. Neurosci. 5 Suppl, 1039-1042 (2002) et B.A.Chizh et al., NMDA receptor antagonists as analgesics: focus on the NR2B subtype, Trends Pharma. Sci. 22, 636-642 (2001)

Partant de là, un problème qui se pose est de disposer d'un traitement ou médicament curatif agissant au niveau du récepteur NMDA en montrant une efficacité dans le traitement des douleurs chroniques, en particulier celles d'origine dysfonctionnelle, mais engendrant par ailleurs peu ou pas d'effets indésirables, c'est-à-dire des effets indésirables limités par rapport à ceux existant lors de l'utilisation des antagonistes des récepteurs NMDA actuels.

Une solution à ce problème est un mélange gazeux contenant une proportion de protoxyde d'azote (N₂O) comprise entre 15 et 45 % en volume pour une utilisation en tant que médicament inhalable pour le traitement curatif d'une douleur chronique d'origine dysfonctionnelle chez un mammifère, par réduction de l'hypersensibilité à la douleur choisie parmi les allodynies et les hyperalgésies.

Le mélange gazeux est administré pendant une durée suffisante pour obtenir une réduction de l'hypersensibilité à la douleur retardée, encore appelée effet anti-sensibilisant retardé, observable au moins 6 heures après la fin de l'inhalation du mélange gazeux par ledit mammifère.

Selon le cas, le mélange gazeux de l'invention peut comprendre l'une ou plusieurs des caractéristiques suivantes :
- le mammifère chez un être humain, à savoir un homme ou une femme, y compris les enfants et les nouveaux-nés.
- la proportion de N₂O dans le mélange gazeux est comprise entre 15 et 40% en volume.
- la proportion de N₂O dans le mélange gazeux est d'au moins 20% en volume, de préférence d'au moins 25% en volume.
- le mélange gazeux est administré pendant une durée suffisante pour obtenir un effet analgésique pendant au moins une partie de la durée d'administration par inhalation du mélange gazeux et un effet anti-sensibilisant retardé après la fin de l'inhalation du mélange gazeux par ledit mammifère.
- le mélange gazeux est administré pendant une durée d'au moins 20 min, de préférence d'au moins 30 min, de préférence d'au moins 40 min, avantageusement d'au moins 1 h.
- le mélange gazeux est administré pendant une durée suffisante pour obtenir un effet anti-sensibilisant retardé se produisant et observable au moins 12 heures après la fin de l'inhalation du mélange gazeux.
- le mélange gazeux est administré pendant une durée suffisante pour obtenir une réduction de l'hypersensibilité à la douleur retardée se produisant et observable au moins 24 heures après la fin de l'inhalation du mélange gazeux.
- le mélange gazeux contient, en outre, entre 20 et 60 % en volume d'oxygène, de préférence entre 20 et 50 % en volume d'oxygène.
- le mélange gazeux est formé de 15 à 45 % en volume de N₂O et au moins 20% en volume d'oxygène.
- le mélange gazeux contient éventuellement un ou plusieurs autres gaz choisis parmi le xénon, l'argon, l'hélium, le néon, le krypton et l'azote, de préférence entre une teneur volumique inférieure à celle du N₂O présent dans le mélange.
- le mélange gazeux est formé de 15 à 45 % en volume de N₂O, au moins 20% en volume d'oxygène, et contient en outre du xénon, de l'argon, de l'hélium, du néon, du krypton ou de l'azote, en particulier de l'azote ou du xénon.
- le mélange gazeux est formé de 15 à 45 % en volume de N₂O, de 21% à 60% en volume d'oxygène, et d'azote pour le reste.
- la douleur d'origine dysfonctionnelle est liée à une fibromyalgie, au syndrome du côlon irritable ou à une céphalée.
- le N₂O peut être par ailleurs administré en combinaison avec un antalgique compris dans les paliers de l'OMS (Organisation Mondiale de la Santé) à savoir :
   - Les *analgésiques du palier I,* aussi appelés "analgésiques périphériques" ou "non morphiniques", qui ont la puissance antalgique la plus faible, par example le paracétamol, l'aspirine, les anti-inflammatoire non-stéroïdiens ...
   - Les *analgésiques du palier II* dits "centraux" ou "morphiniques faibles", car ils sont actifs sur la perception de la douleur au niveau cérébral. Ils sont utilisés seuls, par exemple le tramadol, ou en association avec les analgésiques de palier I, par exemple codéine -paracétamol.
   - Les *analgésiques du palier III* qui regroupent des agonistes morphiniques forts et des agonistes antagonistes.

Dit autrement, l'invention a également trait à une utilisation d'un mélange gazeux contenant du protoxyde d'azote (N₂O) selon l'invention, c'est-à-dire entre 15 et 45% en volume de N₂O tel que décrit ci-avant, pour fabriquer un médicament inhalable pour le traitement curatif d'une douleur chronique chez un mammifère, en particulier chez un être humain, le mélange gazeux étant administré pendant une durée suffisante pour obtenir une réduction de l'hypersensibilité à la douleur retardée , c'est-à-dire un effet anti-sensibilisant retardé, observable au moins 6 heures après la fin de l'inhalation du mélange gazeux par ledit mammifère.

La présente invention va maintenant être mieux comprise grâce à la description suivante et aux exemples qui vont suivre, qui sont donnés à titre purement illustratif.

Dans le cadre de la présente invention, on utilise le protoxyde d'azote (N₂O) à une ou des concentrations préférentiellement comprises entre 15 à 45 % en volume afin de réduire de façon prolongée les douleurs chroniques chez un être humain, c'est un homme, une femme ou un enfant, un nourrisson, principalement celles d'origine dysfonctionnelle.

Le mélange gazeux contenant du protoxyde d'azote (N₂O) utilisable en tant que médicament inhalable pour le traitement curatif d'une douleur chronique chez un patient soufrant d'une telle douleur chronique, peut être administré par inhalation dans le cadre d'une méthode traitement thérapeutique d'un patient soufrant d'une douleur chronique.

Le mélange gazeux de l'invention peut être mis en oeuvre dans le cadre d'une méthode de traitement thérapeutique, dans laquelle on administre ledit mélange gazeux à base de N₂O par inhalation, par exemple au moyen d'un masque respiratoire soit directement relié à une source de N₂O à la concentration requise, par exemple une bouteille de gaz prêt à l'emploi ou alors à la sortie d'un mélangeur de gaz alimenté par plusieurs sources de gaz (O₂, N₂O...) de manière à obtenir le mélange souhaité ; soit relié à un ventilateur respiratoire alimenté en le ou les gaz souhaités.

Typiquement, le mélange gazeux administré est formé essentiellement de protoxyde d'azote, d'oxygène et d'azote, la proportion volumique de protoxyde d'azote étant comprise entre 15 et 45 % et celle d'oxygène étant typiquement comprise entre 20 et 50 %, notamment au moins 21% d'oxygène.

La durée d'administration varie selon les patients entre quelques minutes et plusieurs heures, par exemple entre 5 minutes et 4 ou 5 heures. L'inhalation peut être répétée plusieurs fois de suite, par exemple plusieurs jours de suite.

La concentration et/ou la durée d'administration la plus adaptée à un patient donné peut être choisie de manière empirique par le personnel soignant, par exemple en fonction de l'état de santé ou physique du patient, de la sévérité de la douleur, de son sexe, de son âge...

Le mélange gazeux utilisé selon l'invention est non seulement efficace à faibles concentrations (<50% vol.) pour traiter les hypersensibilités à la douleur, principalement les hyperalgésies et les allodynies, mais aussi n'engendre pas ou peu d'effets indésirables chez les patients.

En effet, le protoxyde d'azote est considéré dans le milieu médical, comme un gaz sûr dès lors que sa concentration est inférieure à environ 50%, comme rappelé par les documents D. Annequin et al. Fixed 50% nitrous oxide oxygen mixture for painful procedures: A French survey. Pediatrics 105, E47 (2000) ; P. Onody, Safety of inhalation of a 50% nitrous eoxide/oxygen premix: a prospective survey of 35 828 administrations, Drug Saf 29, 633-640 (2006); et J.T. Knape, Nitrous oxide not unsafe but used less often, Ned. Tijdschr. Geneeskd. 150, 1053-1054 (2006).

D'autre part, le protoxyde d'azote est un gaz qui possède de fortes propriétés antagonistes des récepteurs N-methyl-D-aspartate receptor (NMDA), comme décrit par le document S.K Georgiev et al., Nitrous oxide inhibits glutamatergic transmission in spinal dorsal horn neurons, Pain 134, 24-31 (2008) ; par le document P. Nagele et al, Nitrous oxide requires the N-methyl-D-aspartate receptor for its action in Caenorhabditis elegans, Proc. Natl. Acad. Sci. U. S. A 101, 8791-8796 (2004), et le document V. Jevtovic-Todorovic, et al., Nitrous oxide (laughing gas) is an NMDA antagonist, neuroprotectant and neurotoxin, Nat. Med. 4, 460-463 (1998).

En outre, des données précliniques consignées dans les documents B. Bessiere. et al., Nitrous oxide prevents latent pain sensitization and long-terma anxiety-like behavior in pain and opioid-experienced rats, Neuropharmacology 53, 733-740 (2007); et P. Richebe et al. Nitrous oxide revisited: evidence for potent antihyperalgesic properties; Anesthesiology 103, 845-854 (2005), ont montré un effet préventif du N₂O sur l'hyperalgésie postopératoire, en particulier celle induite par les opioïdes.

Toutefois, dans le cadre de la présente invention, le mélange gazeux contenant du protoxyde d'azote est non plus utilisé de façon préventive, comme dans le cas de la prévention de l'hyperalgésie post-opératoire, mais de façon curative afin de traiter les patients douloureux chroniques et ainsi obtenir une réduction de leur hypersensibilité à la douleur retardée.

L'utilisation selon l'invention de ce mélange à base de protoxyde d'azote va permettre de réduire ou d'abolir de façon prolongée les hypersensibilités à la douleur, principalement les hyperalgésies et les allodynies, qui caractérisent les patients douloureux chroniques.

Parmi les douleurs chroniques visées par l'invention, on peut citer les douleurs dysfonctionnelles, c'est-à-dire avec absence de lésion connue, comme celles liées à la fibromyalgie, au côlon irritable, aux céphalées...

Au niveau neurobiologique, ces douleurs chroniques sont sous-tendus par un processus de sensibilisation à la douleur qui se traduit par des hyperalgésies et des allodynies intenses et peu sensibles aux composés thérapeutiques actuels.

La présente invention utilisant un mélange gazeux inhalable contenant du protoxyde d'azote vise donc à diminuer ou à d'abolir ces hypersensibilités à la douleur, c'est-à-dire hyperalgésies et allodynies, afin d'améliorer la réhabilitation des patients « douloureux ».

Dit autrement, la présente invention est donc basée sur l'utilisation d'un mélange gazeux thérapeutique contenant moins de 45% en volume de N₂O pour fabriquer un médicament inhalable à effets curatifs destiné à traiter les douleurs chroniques, en particulier les hyperalgésies et allodynies.

Le protoxyde d'azote (N₂O) en une proportion en volumique inférieure à 50% est donc utilisable dans le cadre d'une méthode de traitement thérapeutique, dans laquelle le N₂O est administré par inhalation à un mammifère, en particulier un humain, pour réaliser un traitement curatif d'une douleur chronique chez celui-ci.

### Exemples

Afin de démontrer l'efficacité curative du gaz à base de N₂O selon l'invention, des essais ont été menés avec plusieurs mélanges gazeux contenant différentes concentrations en N₂O, comme donné dans le Tableau 1 ci-dessous.

**Tableau 1**

| Teneurs (% en volume) | N₂O | O₂ | N₂ |
|---|---|---|---|
| Essai 1 (selon l'invention) | 12.5% | 50% | 37.5% |
| Essai 2 (selon l'invention) | 25% | 50% | 25% |
| Essai 3 (selon l'invention) | 35% | 50% | 15% |
| Essai 4 (comparatif) | 50% | 50% | / |

Les essais ont été réalisés de la manière suivante.

### Matériels et Méthodes

On induit chez des rats mâles Sprague-Dawley (8 par groupe), une mononeuropathie par constriction du nerf sciatique. Plus précisément, à J0, les rats sont anesthésiés sous l'halothane et le nerf sciatique (coté patte lésée) est réalisée à l'aide de 4 filament de catgut chromé. Une douleur neuropathique de plus de 40 jours est ainsi induite.

A J7, les rats sont placés dans une enceinte hermétique où ils vont inhaler pendant 75 minutes, le mélange gazeux à tester ou de l'air médical chez les rats contrôles (Sham). Les expositions répétées aux mélanges ont lieu à J7, J8, J9.

D'autres rats sont soumis à des traitements comparatifs avec des produits anti-douleur classiques, à savoir la kétamine (3 × 10 mg/kg, *sc*) et la morphine (1mg/kg, sc).

Ensuite, on opère une mesure du seuil nociceptif via un test de vocalisation en réponse à un stimulus mécanique (Randall-Selitto).

Les données obtenues par mesure sont exprimées en moyenne (± écart-type). Le niveau de significativité est fixé à partir de *P<* 0.05.

### Résultats

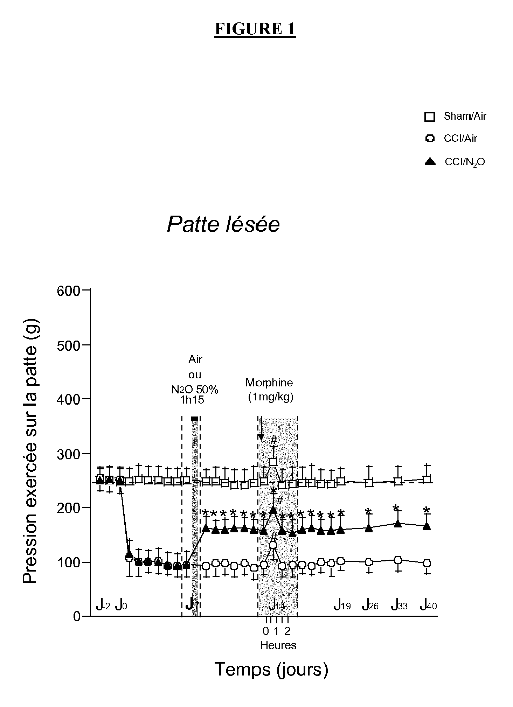

La Figure 1 représente la réduction persistante de l'hypersensibilité à la douleur (∼40%) du côté de la patte lésée suite à un traitement au protoxyde d'azote à 50% en volume (Essai 3).

Au-delà de son effet analgésique bien connu, le protoxyde d'azote à 50 % a permis de réduire chez le rat neuropathique, l'hypersensibilité à la douleur d'environ 40% au niveau de la patte lésée. Cette diminution de l'hypersensibilité à la douleur est observée pendant plus de 30 jours.

A titre comparatif, la morphine, qui est un analgésique opioïde classiquement utilisé en milieu hospitalier, entraine un effet analgésique seulement pendant 30 minutes (J14) sans effet retardé (jours suivant), comme c'est le cas avec le protoxyde d'azote.

L'Essai 3 montre que le traitement avec le mélange gazeux contenant 50% de protoxyde d'azote est nettement plus bénéfique que la morphine sur la réduction des douleurs neuropathiques.

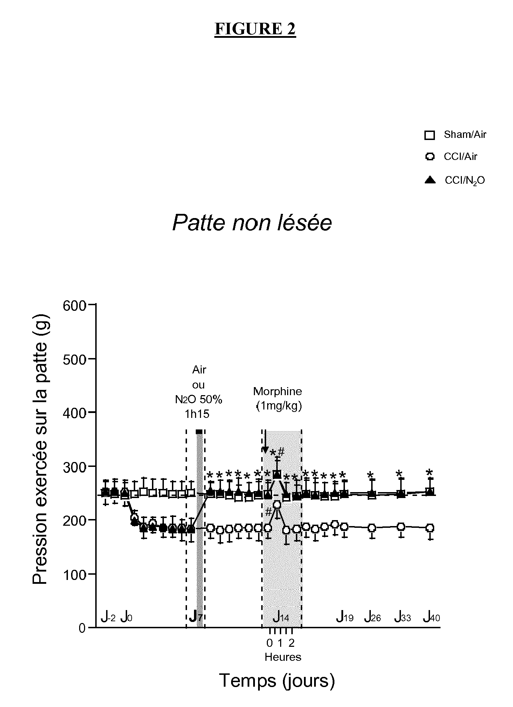

Sur les Figures 1 et 2, les abréviations suivantes sont utilisées :
- Sham : témoin ou contrôle. Ces rats neuropathiques témoins ont subi les mêmes manipulations que les rats neuropathiques (anesthésies, chirurgie, ...) mais pas de ligature du nerf sciatique qui induit la neuropathie. L'objectif est de ne faire varier qu'un seul paramètre à la fois (neuropathie ou non ; respire de l'air ou N2O).
- CCI (pour Chronic Constriction Injury en anglais) : constriction du nerf sciatique. C'est une dénomination bien établie dans la littérature pour désigner le modèle animal neuropathique utilisé (modèle CCI). Dans le cas présent, CCI/N2O désigne le groupe de rats neuropathiques traité au N₂O, alors que CCI/Air désigne le groupe de rats neuropathiques respirant de l'air. Ces deux groupes permettent de déduire l'effet du N₂O chez le rat neuropathique

La Figure 2 montre, quant à elle, l'abolition de l'hypersensibilité à la douleur au niveau de la patte non lésée suite à un traitement au protoxyde d'azote à 50%. Parallèlement à son effet au niveau de la patte lésée, le protoxyde d'azote 50% abolie l'hypersensibilité au niveau de la patte non lésée (controlatérale), ce qui démontre une action centrale du mélange gazeux avec protoxyde d'azote à 50% en volume.

En outre, ces données suggèrent que le N₂O, de part son action au niveau du système central, pourrait réduire l'hypersenbilité à la douleur quelle que soit l'étiologie de la douleur chronique, à savoir douleurs neuropathiques, douleurs dysfonctionnelles, douleurs inflammatoires, du moment qu'une composante sensibilisation centrale soit présente.

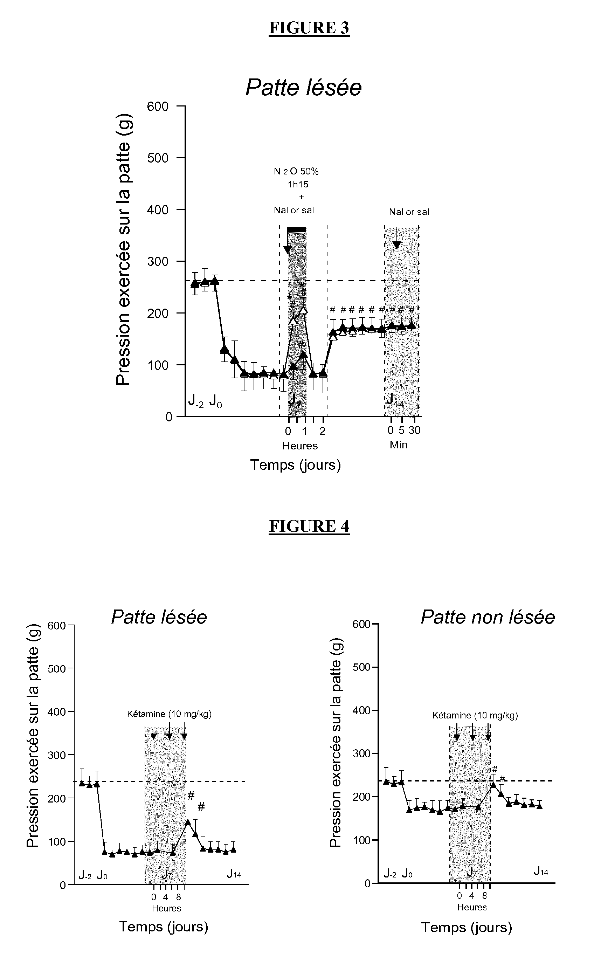

La Figure 3 compare l'effet analgésique à l'effet anti-hyperalgésique du protoxyde d'azote à 50%. Comme on le voit, l'effet anti-hyperalgésique (anti-sensibilisant) du N₂O est indépendant de son effet analgésique. En effet, le blocage de l'effet analgésique du protoxyde d'azote 50% (J7) par la naltrexone qui est un antagoniste des récepteurs opioides, ne modifie pas son effet bénéfique anti-sensibilisant des jours suivant (triangle noir sur Figure 3).

En d'autres termes, l'effet bénéfique retardé, c'est-à-dire durant les jours suivant le traitement au N₂O, et à long-terme du N₂O à 50% est indépendant de son effet analgésique.

Sur la Figure 3, l'abréviation «Nal » est utilisée pour la naltrexone et l'abréviation « Sal » signifie saline.

La Figure 4 compare l'effet du N₂O à 50% à la kétamine qui est un antagoniste des récepteurs NMDA utilisé en milieu hospitalier mais dont l'utilisation reste très limitée chez l'homme de par ses effets indésirables importants, en particulier psychodysleptiques.

Comme on le voit, l'effet de la kétamine est limité à 2 jours comparativement à l'effet prolongé, supérieur à 30 jours, obtenu avec un traitement au N₂O à 50%, lequel ne présente pas par ailleurs les effets indésirables de la kétamine.

Au final, ces données précliniques ont montré un effet inattendu d'un traitement par le protoxyde d'azote à 50% puisque ce gaz est capable de réduire de façon significative et prolongé (> 1 mois) les douleurs chroniques de type neuropathique. Comparativement, l'effet de la morphine est limité à quelques minutes suivant son administration sans aucun effet à long terme anti hyperalgésique. L'effet anti hyperalgésique d'un antagoniste des récepteurs NMDA comme la kétamine est quant à lui limité à 2 jours.

Ainsi, au-delà de son effet analgésique, le N₂O, via ses propriétés anti hyperalgésiques, pourrait représenter une stratégie thérapeutique particulièrement bénéfique dans la prise en charge de patients douloureux chroniques, en particulier chez les patients dont les mécanismes centraux de sensibilisation à la douleur prédominent.

Ces données obtenues avec un mélange gazeux à 50% de N₂O suggèrent en outre que de plus faibles concentrations de N₂O, c'est-à-dire des concentrations inférieures à 50% en volume, devraient présenter les mêmes effets bénéfiques anti-sensibilisants, c'est-à-dire de réduction de l'hypersensibilité à la douleur retardée, tout en réduisant l'effet sédatif/analgésique « aigu » du protoxyde d'azote.

En fait, à des concentrations en N₂O inférieures à 50% en volume, i.e. dites concentrations « sub-anesthésiques », seule une légère amnésie et peu d'effets sédatif et/ou analgésique sont présents chez l'être humain, comme illustré dans le Tableau 2 ci-dessous, ce qui permet une utilisation aisée et sans risque majeur dudit mélange gazeux hors de l'hôpital, notamment à domicile.

**Tableau 2**

| Concentration en N₂O (% vol.) | Effets psychiques | Amnésie | Analgésie | Contact |
|---|---|---|---|---|
| 5 - 25 % | légère sédation | aucune | aucune | présent |
| 26 - 45 % | ébriété | légère | faible | présent |
| 45 - 65 % | somnolence | complète | modérée | présent |
| 66 - 85 % | Patient inconscient | | | |

Afin de démontrer l'efficacité des concentrations en N₂O inférieures à 50% en volume, des essais complémentaires ont été réalisés chez des rats neuropathiques, selon le même protocole que ci-dessus, avec des mélanges gazeux contenant du N₂O, de l'oxygène et éventuellement de l'azote, à savoir (% en volume) :
- mélange A : 12,5 % N₂O + 50% O₂ + 37,5% N₂
- mélange B : 25 % N₂O + 50% O₂ + 25% N₂
- mélange C : 35% N₂O + 50% O₂ + 15% N₂

Les résultats obtenus montrent que l'effet anti-sensibilisant retardé est plus marqué avec le mélange C qu'avec les mélanges A et B. En d'autres termes, il est préférable d'utiliser des concentrations en N₂O supérieures à 15%, avantageusement supérieures à 25% mais inférieures ou égales à 45%. En effet, ces concentrations en N₂O étant inférieures à 50%, les effets sédatifs secondaires néfastes sont nettement moindres qu'avec le mélange à 50% en N₂O testé précédemment, comme rappelé par le Tableau 2 précédent.

En outre, ces essais ont également montré qu'il est avantageux de réaliser plusieurs inhalations successives de N₂O plusieurs jours de suite, par exemple 1 inhalation de 1 heure 15 min pendant 3 jours successifs. En fait, réaliser plusieurs inhalation plusieurs jours d'affilée permet d'augmenter l'effet recherché de réduction de l'hypersensibilité à la douleur retardée.

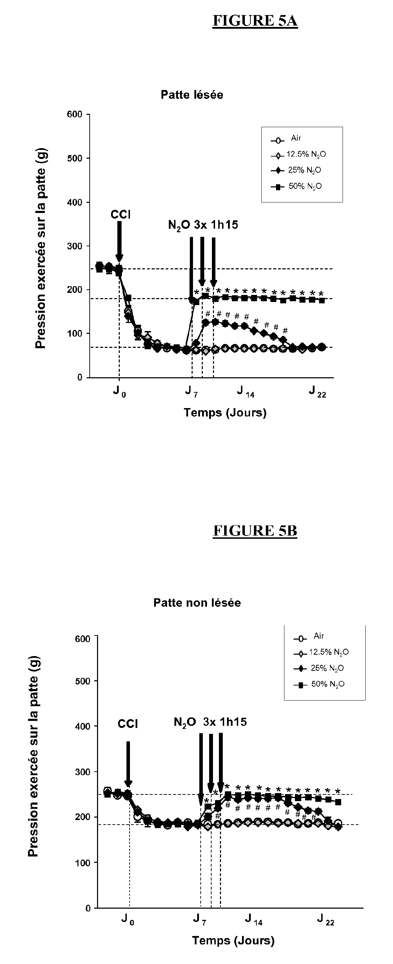

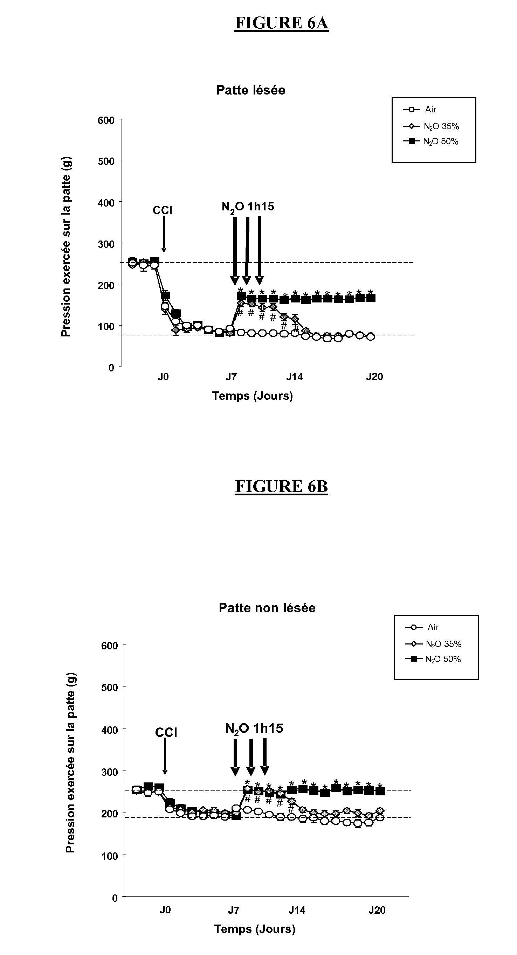

Ceci est illustré sur les Figures 5A, 5B, 6A et 6B qui représentent l'effet anti-sensibilisant retardé des mélanges A, B et C ainsi que d'un mélange à 50% de N₂O et d'air (témoin) sur une patte de rat lésée par constriction lâche (CCI) du nerf sciatique (FIG. 5A, 6A) et sur une patte de rat non lesée (FIG. 5B, 6B), sur lesquelles sont appliquées des pressions mécaniques afin de mesurer le seuil de douleur.

Comme on le voit, administrer le N₂O, 3 fois de suite (1 administration par jour) pendant 1h 15min à chaque inhalation, permet d'obtenir une réduction de l'hypersensibilité à la douleur retardée de longue durée, se produisant et observable environ 24h après la première inhalation et continuant à se produire pendant près de 1 semaines après la dernière inhalation pour le mélange B, C et le mélange à 50%. Par contre, le mélange A (12,5% N₂O) n'a pas montré dans ce cas de différence significative avec le témoin (air), ce qui confirme qu'il est préférable d'utiliser des concentrations de N₂O d'au moins 15% en volume chez le rat.

De plus, ces essais ont également montré qu'il est avantageux de réaliser une inhalation supérieure à 30 min de N₂O par exemple 1h. En fait, réaliser une inhalation de plus de 30 min permet d'augmenter l'effet anti-sensibilisant recherché.

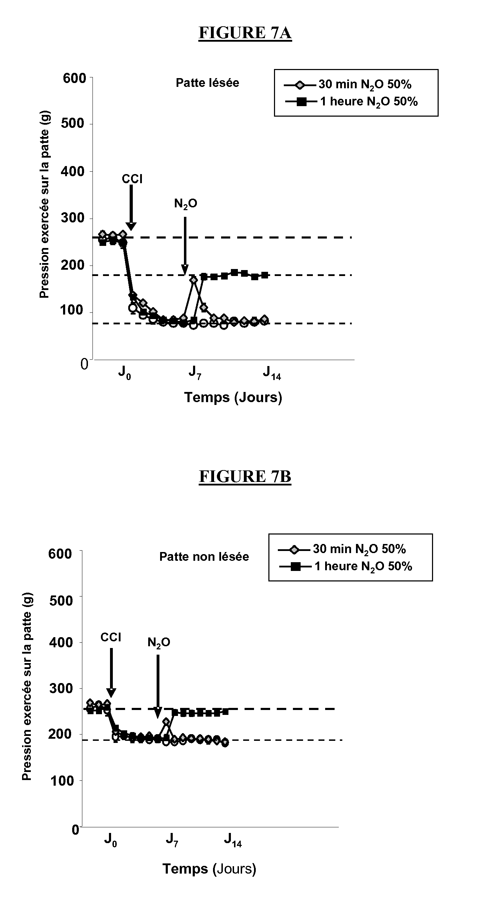

Ceci est illustré sur les Figures 7A et 7B d'un mélange à 50% de N₂O et d'air (témoin) sur une patte de rat lésée par constriction lâche (CCI) du nerf sciatique (FIG. 7A) et sur une patte de rat non lésée (FIG. 7B), sur lesquelles sont appliquées des pressions mécaniques afin de mesurer le seuil de douleur.

Comme on le voit, administrer le N₂O, pendant 1h permet d'obtenir une réduction de l'hypersensibilité à la douleur retardée, c'est-à-dire un effet anti-sensibilisant, de longue durée, se produisant et observable environ 24h après la première inhalation et continuant à se produire pendant près de 1 semaines après la dernière inhalation pour un mélange à 50%.

De là, selon l'invention, on utilise préférentiellement un mélange gazeux contenant du protoxyde d'azote (N₂O) en une proportion en volumique inférieure à 50%, typiquement inférieure à 48%, avantageusement comprise entre 15 et 45 % en volume, en tant que médicament inhalable pour le traitement curatif d'une douleur chronique chez un mammifère, le mélange gazeux étant administré pendant une durée suffisante pour obtenir une réduction de l'hypersensibilité à la douleur retardée, c'est-à-dire un effet anti-sensibilisant retardé, observable au moins 6 heures, en général d'au moins 12 à 24 heures, après la fin de l'inhalation du mélange gazeux par ledit mammifère, en particulier chez l'être humain.

## Revendications

1. Mélange gazeux contenant une proportion de protoxyde d'azote (N₂O) comprise entre 15 et 45 % en volume pour une utilisation en tant que médicament inhalable pour le traitement curatif d'une douleur chronique d'origine dysfonctionnelle chez un mammifère, par réduction de l'hypersensibilité à la douleur choisie parmi les allodynies et les hyperalgésies.

2. Mélange gazeux selon la revendication précédente, **caractérisé en ce que** la douleur d'origine dysfonctionnelle est liée à une fibromyalgie, au syndrome du côlon irritable ou à une céphalée.

3. Mélange gazeux selon l'une des revendications précédentes, **caractérisé en ce que** la proportion de N₂O dans le mélange gazeux est d'au moins 20% en volume, de préférence d'au moins 25% en volume.

4. Mélange gazeux selon l'une des revendications précédentes, **caractérisé en ce que** le mélange gazeux est administré pendant une durée suffisante pour obtenir un effet analgésique pendant au moins une partie de la durée d'administration par inhalation du mélange gazeux et un effet anti-sensibilisant retardé après la fin de l'inhalation du mélange gazeux par ledit mammifère.

5. Mélange gazeux selon la revendication 4, **caractérisé en ce que** le mélange gazeux est administré pendant une durée d'au moins 20 min, de préférence d'au moins 30 min.

6. Mélange gazeux selon la revendication 4, **caractérisé en ce que** le mélange gazeux est administré pendant une durée suffisante pour obtenir un effet anti-sensibilisant retardé se produisant et observable au moins 12 heures après la fin de l'inhalation du mélange gazeux.

7. Mélange gazeux selon l'une des revendications précédentes, **caractérisé en ce que** le mélange gazeux est administré pendant une durée suffisante pour obtenir un effet anti-sensibilisant retardé se produisant et observable au moins 24 heures après la fin de l'inhalation du mélange gazeux.

8. Mélange gazeux selon l'une des revendications précédentes, **caractérisé en ce que** le mélange gazeux contient, en outre, entre 20 et 60 % en volume d'oxygène.

9. Mélange gazeux selon l'une des revendications précédentes, **caractérisé en ce que** le mélange gazeux est formé de 15 à 45 % en volume de N₂O et au moins 20% en volume d'oxygène, et contient du xénon, de l'argon, de l'hélium, du néon, du krypton et de l'azote.

10. Mélange gazeux selon l'une des revendications précédentes, **caractérisé en ce que** le mélange gazeux est formé de 15 à 45 % en volume de N₂O, de 21% à 60% en volume d'oxygène, et d'azote pour le reste.

11. Mélange gazeux selon l'une des revendications précédentes, **caractérisé en ce que** le mammifère est un être humain.

12. Mélange gazeux selon l'une des revendications précédentes, **caractérisé en ce qu'**il est administré en combinaison avec un antalgique.
